# EUROPEAN PATENT SPECIFICATION

(11) **EP 2 991 545 B1**
(45) Date of publication and mention of the grant of the patent: **30.11.2022**
(21) Application number: 14744617.3
(22) Date of filing: 01.05.2014
(51) Int. Cl.: H04N 9/31, A61B 5/00, A61B 5/16, A61B 5/11, G16H 20/70

(54) **APPARATUS FOR USE IN THE PERFORMANCE OF COGNITIVE BEHAVIOUR THERAPY**
VORRICHTUNG ZUR VERWENDUNG IN DER KOGNITIVEN VERHALTENSTHERAPIE
APPAREIL DESTINÉ À ÊTRE UTILISÉ DANS LA RÉALISATION D'UNE THÉRAPIE DE COMPORTEMENT COGNITIF

(30) Priority: 01.05.2013 GB 201307896
(43) Date of publication of application: 09.03.2016
(73) Proprietor: Third Eye Neurotech Ltd, Morpeth Northumberland NE61 1PY (GB)
(72) Inventor: PARR, Jeremy, Newcastle-upon-Tyne Tyne and Wear NE1 4LP (GB); McCONACHIE, Helen, Queen Victoria Road Newcastle Tyne and Wear NE1 4LP (GB); SMITH, Paul, Newcastle-upon-Tyne Tyne and Wear NE15 8DE (GB)
(74) Representative: Elsworth, Dominic Stephen
(86) International application number: PCT/GB2014/051355
(87) International publication number: WO 2014/177875

(56) References cited:
- WO-A1-99/06981
- WO-A1-2007/114404
- US-A1- 2003 131 351
- US-A1- 2008 043 097
- US-B1- 6 425 764

## Description

### Field of the Invention

The present invention relates to enhancing the conduct of cognitive behaviour therapy and in particular to an apparatus for use in such therapy.

### Background of the Invention

Cognitive behaviour therapy is widely used. In relation to individuals, particularly people with autism spectrum disorder, cognitive behaviour therapy may be used to assist individuals in becoming accustomed to activities which would otherwise present significant challenges in their daily lives, in particular to overcoming phobias.

For example, a child may find crossing the road, or speaking to a person in a shop extremely difficult. Another child may have a particular fear, for example, of pigeons which restricts their willingness to go out of the house.

It is well documented that such problems may be treated by gradually increasing the challenge posed to the individual, in the context of helping the individual develop strategies for keeping relaxed and gaining confidence.

A known method of attempting to assist an individual with a problem of the type described above includes a psychologist or therapist accompanying the individual to the environment in which the particular problem occurs and increasing the challenge to the individual by small incremental steps.

The psychologist or therapist attempts to control the challenge posed to the individual in the environment. However, this is very difficult to achieve because the real world the environment is in many was not controllable. For example, in the case of an individual who finds going to the till in a shop challenging, the psychologist or therapist may be able to control certain aspects of the individual's experience, for example which shop is entered, when the person at the till is approached, etc. However, the therapist cannot control other shoppers, or the reaction of the person at the till. To overcome difficulties in arranging graded steps in exposure, the therapist may help the individual to imagine scenes in a graded way. However, for individuals with autism control of imagination may be particularly difficult.

It would be desirable to be able to accustom individuals to situations in a more controlled environment. This would not only allow the individuals to progress more rapidly in overcoming problems, but may also relieve pressure on resources and allow more individuals to be treated.

However, in order for an individual to become accustomed to a situation, the individual must believe that the controlled environment is believable as being representative of the problem. For example, looking at pictures in a book or on a computer screen is unlikely to accustom the individual to the problem sufficiently for the individual to then go out into the real world and face the problem.

Surprisingly, it has been found that by exposing individuals to a progressively increasing challenge within a surround vision environment, individuals may overcome their phobias, with transfer of confidence to real life. An apparatus for virtual reality immersion therapy for treating psychological, psychiatric, medical, educational and self-help problems is described in US6425764 B1.

### Summary of the Invention

According to the invention there is provided an apparatus for use in the performance of cognitive behaviour therapy according to claim 1. Preferred embodiments of the invention are defined by dependent claims 2-14.

### Brief Decription of the Drawings

In the drawings, which illustrate preferred embodiments of the apparatus for use in the performance of cognitive behaviour therapy:
Figure 1 is a schematic representation of apparatus according to the invention;
Figure 2 is a schematic illustration of a camera used in the generation of images for projection into the apparatus illustrated in Figure 1; and
Figure 3a is a schematic representation of the apparatus in use;
Figure 3b is a side view of the apparatus illustrated in Figure 3a;
Figure 3c is a front view of the apparatus illustrated in Figure 3b; and
Figure 3d is a view of a viewing screen.

### Detailed Description of the Preferred Embodiment and Examples

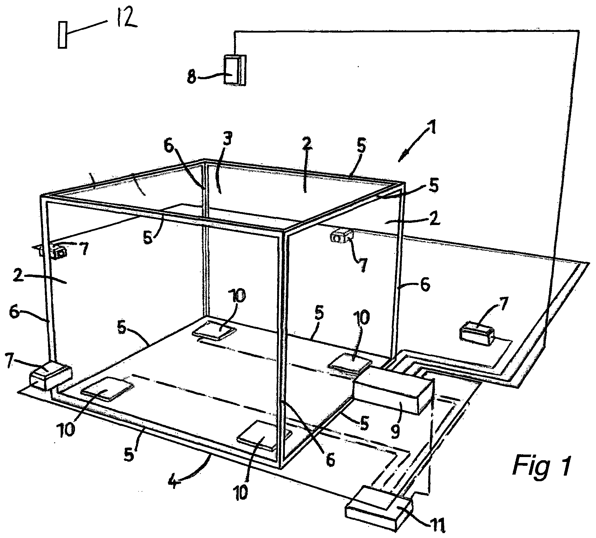

Referring now to Figure 1, there is shown apparatus including an enclosure 1 comprising four projection screens 2 forming the walls of the enclosure 1, a fifth projection screen 3 constituting the enclosure's ceiling, and a floor 4. Each projection screen 2 is attached to horizontal and vertical frame members 5, 6. The projection screen 3 is attached to the upper horizontal frame member 5, whilst the floor 4 is attached to the lower horizontal frame members 5. The result is a completely enclosed space. At least one of the screens 2 is either moveable or includes a closable access, thereby permitting a person to enter the enclosure 1.

In this example, four video projectors 7 are arranged outside the enclosure 1 and are aligned to project images onto the screens 2. A fifth video projector 8 is located above the screen 3 and is aligned to project images onto the screen 3. Each projector 7, 8 projects an image onto one of the screens 2, 3, respective projected images each filling one of the screens 2, 3.

As an alternative, the screens 2, 3 and projectors 7, 8 may both be replaced by flat panel_display or screen technology, such as plasma or LCD screens.

The projection of images onto the screens 2, 3 is controlled by a controller in the form of one or more processors (in the example one processor 9), which process the video and audio streams.

The floor 4 of the room 1 includes loud speakers 10, one in each corner of the floor 4. The delivery of sound to the loudspeakers 10 is controlled by the processor 9, and is fed through an amplifier 11 to ensure adequate loudness within the room 1.

The processor 9 synchronizes the transmission of images to the projectors 7, 8. In one method of synchronization the processor identifies time stamps in the frames of recorded data. If the image projected by one of the projectors is more than 1/100^{th} second out of synchronization with the images projected from the other projectors, the processor executes a synchronization routine (in this case image frames are transmitted at 1/100^{th} second intervals. If image frames are transmitted at different intervals, such as 1/50^{th} second intervals, then the processor is arranged to execute its synchronization routine if the transmission of images by one of the projectors is out by more than the transmission rate, of in the latter case 1/50^{th} second). This is important, since poor synchronization leads to a person in the enclosure 1 losing his point of reference. Rather than using the processor 9 to perform the synchronization of images projected onto the screens, the image data may be fed to the processor and hence the projectors in a synchronous stream.

Another function of the processor 9 is to synchronize the transmission of images via the projectors with the transmission of sound via the amplifier 11 and loudspeakers 10. The processor is adapted to control the transmission of sound, and the loudness of that sound to any one of the speakers 10. This serves two purposes. First, sound can be properly associated with the images projected on the screens 2, 3, for example where the image is of a vehicle the sound and loudness of that sound from any of the speakers 10 changes so that the sound appears to emanate from the vehicle, where-ever it is on the screens 2, 3. Second, where interactivity is provided (described in greater detail below), the processor 9 can direct sound at a desired loudness to one or more of the screens on which images are projected as a result of an interactive act.

A command device in the form of a remote control 12 is configured to communicate with the processor 9 in order to determine the images projected on the screens 2, 3.

The apparatus may include an anxiety monitoring device, which may be arranged to communicate with the processor 9. The scenes may have information associated with them indicative of level of anxiety likely to be caused. The processor 9 may be configured to override an input of the command device where the scene that would be projected on to the screens is indicated as causing anxiety significantly different from a threshold level sensed by the anxiety monitoring device.

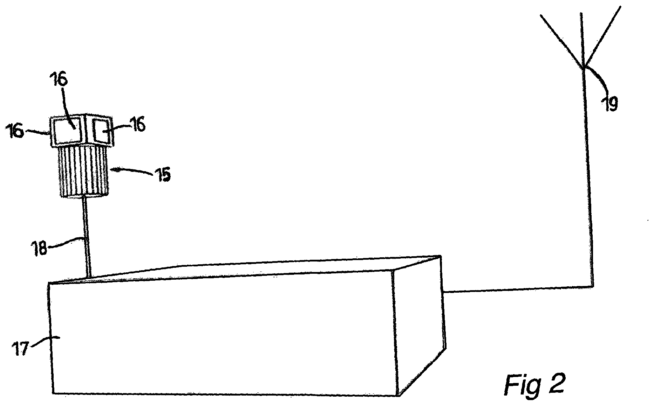

Figure 2 illustrates a digital camera 15 comprising four camera heads 16 (two heads being fully visible in Figure 2). Digital signals from the camera heads 16 are transmitted to a digital video recorder 17 by connection 18. The digital video recorder 17 may retransmit the recorded signals via a transmission antenna 19. Each camera head 16 includes a lens, and a digital video processor. A compression unit may also be included, but is not essential. Signals produced by the digital video processors may be transmitted to the digital video recorder either by cable, or a radio signal. Where transmission is by a radio signal, the camera is provided with a common processor that receives output signals from the digital video processors. The common processor converts the digital video signals into a radio signal and transmits this to the video recorder 17. Where a cable is used, a common processor is not required, the digital video signals being transmitted directly from the digital video processors to the video recorder.

The images projected via projectors 7, 8 may be computer generated images or images recorded using a camera, for example a camera of the type illustrated in Figure 2.

The system provides one immersive image extending 360 degrees around the screens 2. To achieve this, the field of view of each camera head 16 must be at least 90 degrees (the lens shooting footage for the ceiling screen needs to shoot at not less than 121 degrees). The desired image can be achieved either by matching the image taken by the camera to the shape of the screen 2 on to which it is to be projected, or by processing the images to fit the screens. In order to remove aberrations and distortions advanced image processing techniques may be employed such as correction for alignment distortions, perspective distortions, pincushion distortions, barrel distortions. Whereas one camera may be employed for each screen, equally a number of cameras may be employed to capture the scene to be projected onto any one screen, with the images from each camera being stitched together to provide an apparently seamless image for projection onto a screen.

The images projected onto the screens may be interactive. To provide for interactivity, it is necessary to include in the apparatus certain input devices, such as gyroscopic sensors, touch sensitive screens, movement sensors for sensing movement of people within the enclosure.

In use, the person receiving therapy is located in the room 1. A therapist selects the images to be displayed on the screens 2, 3, and using the remote control 12 may perform the usual commands that are associated with the projection of images, i.e. play, fast forward, rewind, select from an image library, etc. The therapist is situated in the room 1. The apparatus may include communication means to permit communication between the therapist and the person receiving therapy where the therapist is situated outside the room 1. The remote control may be comprised in a tablet computer for example.

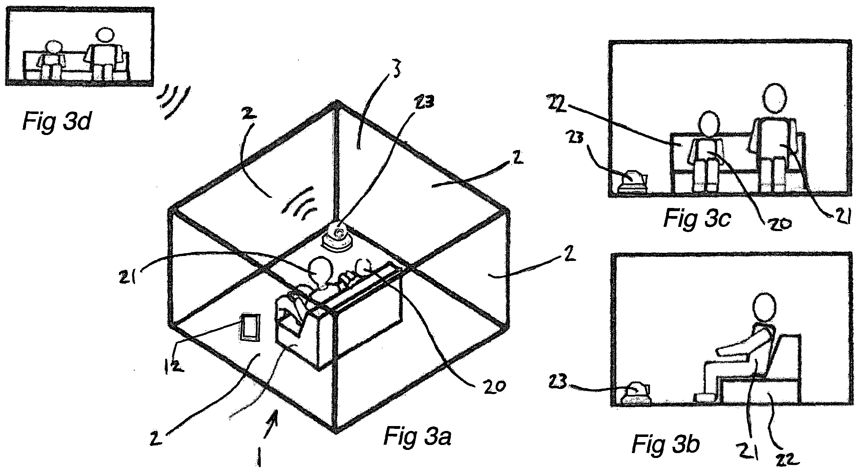

Figures 3a to 3d best illustrate use of the apparatus. In Figures 3a to 3b it can be seen that the patient 20 and the therapist 21 are both seated on a sofa 22 within the enclosure 1. The enclosure 1 is as described with reference to Figure 1. The remote control may be used either by the patient 20 or the therapist 21, as directed by the therapist.

It can be seen that a camera 23 is located in the enclosure 1. The purpose of the camera is to capture the behaviour of the patient and therapist during therapy. The camera 23 is a web cam in the present example.

Figure 3d illustrates a screen 24 such as a visual display unit. The pictures from the camera 23 are displayed live on the screen 24. In the example, the screen 24 is situated in a room separate from the enclosure 1. One purpose of the screen 24 is to allow observation of the therapy within the enclosure 1 to be observed by the patient's parents or carers. Another purpose of the screen 24 is to allow researchers and other professionals to observe the behaviour of the patient and therapist during therapy.

### Examples

The apparatus of the invention was used with five children as described in detail below:
Each child received four 20-30 minute sessions in the apparatus. Two sessions took place during one visit with a 15 minute break in the middle. A therapist was in the room with the child the whole time. The therapist regularly checked in with the child about their level of anxiety on the visual scale and also coached them in relaxation techniques and coping self-talk.

Relaxing scenes were generic and included a dolphin scene, a field scene with relaxing, swaying grass and a colourful balls scene where balls pile up and then crash to the ground. Participants were allowed to choose which scene they wanted.

### Participant 1

12 year old with diagnosis of Asperger's syndrome and Dyspraxia.
Issue: unable to cross bridges, particularly if water underneath
Scene: Recreation of the Armstrong Bridge in Jesmond. Scene was developed which allowed the water level underneath the virtual bridge to be gradually increased and also to increase the height of the bridge in three increments.

After four sessions the participant lost their fear of crossing bridges. In real life he is now able to cross any bridge. He is also now able to do rock climbing and to be able to climb stairs e.g. in a multi - story car park.

### Participant 2

12 year old with diagnosis of autism spectrum disorder.
Issue: phobia of pigeons
Scene: playground scene where the number of pigeons was gradually increased, both flying in and taking off

After four sessions able to do many things he couldn't previously - sit by a window in his house, walk past pigeons at entrance to shopping mall, no longer has panic attacks.

### Participant 3

8 year old with a diagnosis of Asperger's syndrome.
Issue: Following a minor car accident where his grandmother was driving, the participant had developed a phobia of being a passenger in a car when a woman was driving.
Scene: A virtual car was developed, which looked very similar to his grandmothers car. He virtually got in the car with the therapist and together they drove through a city scene.
After two sessions the child had overcome his phobia and when he arrived at the Blue Room for his second visit he told us he had been out driving with both his grandmother and mother.

### Participant 4

11 year old with a diagnosis of Asperger's syndrome.
Issue: Social phobia around shopping
Scene: Petrol station kiosk where the target was to pick up a newspaper and talk with a virtual shop assistant. Gradually the amount of speaking to the shop assistant was increased.

After four sessions the participant was able to buy a newspaper in real life. He then progressed to shopping in a shopping mall with his father waiting inside the shop. Three months post Blue Room sessions he was shopping independently with friends.

### Participant 5

9 year old with a diagnosis of autism spectrum disorder.
Issue: afraid to travel on crowded buses. Parents did not drive so this caused major problems for the family if they needed to get anywhere
Scene: Bus stop with shelter. Bus arrives and stops and the number of people on the bus is gradually increased. Participant virtually gets on bus with therapist.

After four sessions the participant was no longer afraid of crowded buses and could travel with his family without incident. This was translated to metros and he is now able to travel on crowded metros as well.

## Claims

1. An apparatus for use in the conduct of cognitive behaviour therapy, the apparatus comprising:
a surround vision system comprising:
an enclosure (1) including a plurality of walls (6), a ceiling (5) and a floor (4), the enclosure providing a completely enclosed space, and within the enclosure a position for both an individual receiving therapy and a therapist to be situated during provision of therapy and a camera (23), wherein each of the plurality of walls (6) and the ceiling (5) are formed by a respective screen (2, 3), wherein one of the respective screens provides a closable access to and from the enclosure; and
at least one processor (9) and either a plurality of projectors (7, 8) configured to project images onto the respective screens (2, 3) or the respective screens are display screens configured to display images thereon such that the said images may be viewed from within the completely enclosed space, wherein the at least one processor (9) synchronises the projection of images onto each screen (2, 3) or the display of images on each display screens such that the images are perceived by the viewer as being uninterrupted;
sets of recorded scenes each set relating to the same theme, each set configured to represent a different level of challenge for the same theme;
a controller for controlling the displaying images of a set on the screens of the surround vision system; and
a command device (12) adapted to communicate with, and send control signals to the controller, the command device for use by a person overseeing the delivery of therapy to the individual or by the individual receiving therapy for controlling the display of scenes on the screens of the surround vision system
wherein the camera (23) located within the enclosure (1) is configured to capture the behaviour of the patient and therapist during use of the apparatus.

2. Apparatus according to Claim 1, wherein the surround vision system includes a sound system (10, 11) configured to play and emit sound synchronised with the images shown on the screens of the system.

3. Apparatus according to Claim 1 or 2, further including one or more anxiety monitoring devices.

4. Apparatus according to Claim 3, wherein the or each anxiety monitoring device is in communication with the controller.

5. Apparatus according to Claim 3 or 4, wherein the anxiety monitoring devices include one or more of: a heart rate monitor, blood pressure sensor, skin temperature sensor and an eye tracker.

6. Apparatus according to any preceding claim, wherein the command device comprises a hand held remote control (12).

7. Apparatus according to any preceding claim, wherein the command device is adapted to interact with a screen of the surround vision apparatus.

8. Apparatus according to any preceding claim, wherein the command device comprises a part of an eye tracking system.

9. Apparatus according to any preceding claim, wherein the command device includes a glove adapted to interact with a screen of the surround vision system.

10. Apparatus according to any of Claims 3 to 5, or 6 to 9, wherein the controller is configured to over-ride the command device when an output from the anxiety monitoring device exceeds a threshold value.

11. Apparatus according to any of Claims 3 to 5, or 6 to 10 when dependent on Claim 3, wherein the controller is configured to record the scene displayed, the commands given via the command device and the output of the or each anxiety monitoring device.

12. Apparatus according to any preceding claim, further including a library of sets of scenes, each set of different a scenario or a different level of challenge for the same scenario.

13. Apparatus according to Claim 12, when the library of sets of scenes further includes a set of scenes configured to cause the individual receiving therapy to relax.

14. Apparatus according to any preceding claim, wherein the apparatus further comprises a display screen situated outside the enclosure and wherein the camera located within the enclosure and configured to capture the behaviour of the patient and therapist during use of the apparatus is connected to the display screen situated outside the enclosure for display of images captured by said camera on said display screen in real time.

## Patentansprüche

1. Vorrichtung zur Verwendung bei der Durchführung einer kognitiven Verhaltenstherapie, wobei die Vorrichtung Folgendes umfasst:
ein Rundumsichtsystem, das Folgendes umfasst:
ein Gehäuse (1) mit mehreren Wänden (6), einer Decke (5) und einem Boden (4), wobei das Gehäuse einen vollständig umschlossenen Raum und innerhalb des Gehäuses eine Position, in der sich sowohl eine Person in Therapie als auch ein Therapeut während der Durchführung der Therapie aufhalten können, und eine Kamera bereitstellt, wobei jede der mehreren Wände (6) und die Decke (5) durch einen jeweiligen Bildschirm (2, 3) gebildet werden, wobei einer der jeweiligen Bildschirme einen verschließbaren Zugang zu und von dem Gehäuse bereitstellt; und
mindestens einen Prozessor (9) und entweder mehrere Projektoren (7, 8), die zum Projizieren von Bildern auf die jeweiligen Bildschirme (2, 3) konfiguriert sind, oder die jeweiligen Bildschirme sind Anzeigebildschirme, die zum Anzeigen von Bildern darauf konfiguriert sind, so dass die genannten Bilder von innerhalb des vollständig umschlossenen Raums betrachtet werden können, wobei der mindestens eine Prozessor (9) die Projektion von Bildern auf jeden Bildschirm (2, 3) oder die Anzeige von Bildern auf jedem Anzeigebildschirm so synchronisiert, dass die Bilder vom Betrachter als ununterbrochen wahrgenommen werden;
Sätze von aufgezeichneten Szenen, wobei sich jeder Satz auf dasselbe Thema bezieht und jeder Satz zum Darstellen eines unterschiedlichen Herausforderungsniveaus für dasselbe Thema konfiguriert ist;
eine Steuerung zum Steuern der Anzeige von Bildern eines Satzes auf den Bildschirmen des Rundumsichtsystems; und
ein Befehlsgerät (12), so ausgelegt, dass es mit der Steuerung kommuniziert und Steuersignale an diese sendet, wobei das Befehlsgerät von einer die Durchführung der Therapie an der Person überwachenden Person oder von der die Therapie erhaltenden Person zum Steuern der Anzeige von Szenen auf den Bildschirmen des Rundumsichtsystems benutzt wird,
wobei die innerhalb des Gehäuses (1) befindliche Kamera (23) zum Erfassen des Verhaltens des Patienten und des Therapeuten während der Benutzung der Vorrichtung konfiguriert ist.

2. Vorrichtung nach Anspruch 1, wobei das Rundumsichtsystem ein Tonsystem (10, 11) beinhaltet, das zum Abspielen und Ausgeben von Ton konfiguriert ist, der mit den auf den Bildschirmen des Systems angezeigten Bildern synchronisiert ist.

3. Vorrichtung nach Anspruch 1 oder 2, die ferner eine oder mehrere Angstzustandsüberwachungsgeräte enthält.

4. Vorrichtung nach Anspruch 3, wobei das oder jedes Angstzustandsüberwachungsgerät mit der Steuerung in Verbindung ist.

5. Vorrichtung nach Anspruch 3 oder 4, wobei die Angstzustandsüberwachungsgeräte eines oder mehrere von einem Herzfrequenzsensor, einem Blutdrucksensor, einem Hauttemperatursensor und einem Eyetracker umfassen.

6. Vorrichtung nach einem vorherigen Anspruch, wobei das Befehlsgerät eine Handfernbedienung (12) umfasst.

7. Vorrichtung nach einem vorherigen Anspruch, wobei das Befehlsgerät zum Interagieren mit einem Bildschirm der Rundumsichtvorrichtung ausgelegt ist.

8. Vorrichtung nach einem vorherigen Anspruch, wobei das Befehlsgerät einen Teil eines Eyetracker-Systems umfasst.

9. Vorrichtung nach einem vorherigen Anspruch, wobei das Befehlsgerät einen Handschuh beinhaltet, der mit einem Bildschirm des Rundumsichtsystems interagieren kann.

10. Vorrichtung nach einem der Ansprüche 3 bis 5 oder 6 bis 9, wobei die Steuerung zum Übersteuern des Befehlsgeräts konfiguriert ist, wenn ein Ausgang vom Angstzustandsüberwachungsgerät einen Schwellenwert übersteigt.

11. Vorrichtung nach einem der Ansprüche 3 bis 5 oder 6 bis 10 wenn von Anspruch 3 abhängig, wobei die Steuerung zum Aufzeichnen der angezeigten Szene, der über das Befehlsgerät erteilten Befehle und des Ausgangs des oder jedes Angstzustandsüberwachungsgeräts konfiguriert ist.

12. Vorrichtung nach einem vorherigen Anspruch, ferner mit einer Bibliothek von Szenensätzen, wobei jeder Satz ein anderes Szenario oder ein anderes Herausforderungsniveau für dasselbe Szenario enthält.

13. Vorrichtung nach Anspruch 12, wenn die Bibliothek von Szenensätzen außerdem einen Satz von Szenen enthält, die so konfiguriert sind, dass sie die zu behandelnde Person zur Entspannung veranlassen.

14. Vorrichtung nach einem vorherigen Anspruch, wobei die Vorrichtung ferner einen außerhalb des Gehäuses befindlichen Anzeigebildschirm umfasst und wobei die Kamera, die sich innerhalb des Gehäuses befindet und zum Erfassen des Verhaltens des Patienten und des Therapeuten während der Benutzung der Vorrichtung konfiguriert ist, mit dem außerhalb des Gehäuses befindlichen Anzeigebildschirm verbunden ist, um die von der genannten Kamera erfassten Bilder auf dem genannten Anzeigebildschirm in Echtzeit anzuzeigen.

## Revendications

1. Appareil servant à des fins d'utilisation dans la conduite d'une thérapie cognitive du comportement, l'appareil comportant :
un système de vision périphérique comportant :
une enceinte (1) comprenant une pluralité de parois (6), un plafond (5) et un plancher (4), l'enceinte mettant en œuvre un espace entièrement fermé, et à l'intérieur de l'enceinte une position pour à la fois un individu recevant la thérapie et un thérapeute à des fins de positionnement au cours de la réalisation de la thérapie et une caméra (23),
dans lequel chacune de la pluralité de parois (6) et le plafond (5) sont formés par un écran respectif (2, 3),
dans lequel l'un des écrans respectifs fournit un accès en mesure d'être fermé vers et en provenance de l'enceinte ; et
au moins un processeur (9) et soit une pluralité de projecteurs (7, 8) sont configurés pour projeter des images sur les écrans respectifs (2, 3), soit les écrans respectifs sont des écrans d'affichage configurés pour afficher des images sur ceuxci, de telle sorte que lesdites images peuvent être visualisées depuis l'intérieur de l'espace entièrement fermé, dans lequel ledit au moins un processeur (9) synchronise la projection d'images sur chaque écran (2, 3) ou l'affichage d'images sur chaque écran d'affichage de telle sorte que les images sont perçues par celui qui regarde comme étant ininterrompues ;
des ensembles de scènes enregistrées, chaque ensemble se rapportant au même thème, chaque ensemble étant configuré pour représenter un niveau différent de défi pour le même thème ;
un système de commande servant à contrôler les images d'affichage d'un ensemble sur les écrans du système de vision périphérique ; et
un dispositif d'instructions (12) adapté pour communiquer avec le système de commande, et pour envoyer des signaux de commande à celui-ci, le dispositif d'instructions servant à des fins d'utilisation par une personne qui supervise la réalisation de la thérapie de l'individu ou par l'individu qui reçoit la thérapie pour contrôler l'affichage de scènes sur les écrans du système de vision périphérique ;
dans lequel la caméra (23) située à l'intérieur de l'enceinte (1) est configurée pour capturer le comportement du patient et du thérapeute au cours de l'utilisation de l'appareil.

2. Appareil selon la revendication 1, dans lequel le système de vision périphérique comprend un système audio (10, 11) configuré pour jouer et émettre du son de manière synchronisée avec les images affichées sur les écrans du système.

3. Appareil selon la revendication 1 ou la revendication 2, comprenant par ailleurs un ou plusieurs dispositifs de surveillance de l'anxiété.

4. Appareil selon la revendication 3, dans lequel le ou chaque dispositif de surveillance de l'anxiété est en communication avec le système de commande.

5. Appareil selon la revendication 3 ou la revendication 4, dans lequel les dispositifs de surveillance de l'anxiété comprennent un ou plusieurs parmi : un moniteur de la fréquence cardiaque, un détecteur de la pression artérielle, un capteur de la température corporelle, et un suiveur oculaire.

6. Appareil selon l'une quelconque des revendications précédentes, dans lequel le dispositif d'instructions comporte une télécommande portative (12).

7. Appareil selon l'une quelconque des revendications précédentes, dans lequel le dispositif d'instructions est adapté à des fins d'interaction avec un écran de l'appareil de vision périphérique.

8. Appareil selon l'une quelconque des revendications précédentes, dans lequel le dispositif d'instructions comporte une partie d'un système d'oculométrie.

9. Appareil selon l'une quelconque des revendications précédentes, dans lequel le dispositif d'instructions comprend un gant adapté à des fins d'interaction avec un écran du système de vision périphérique.

10. Appareil selon l'une quelconque des revendications 3 à 5, ou des revendications 6 à 9, dans lequel le système de commande est configuré pour prendre la priorité sur le dispositif d'instructions quand une sortie en provenance du dispositif de surveillance de l'anxiété dépasse une valeur de seuil.

11. Appareil selon l'une quelconque des revendications 3 à 5, ou des revendications 6 à 10, quand dépendantes de la revendication 3, dans lequel le système de commande est configuré pour enregistrer la scène affichée, les instructions données par le biais du dispositif d'instructions et la sortie en provenance du ou de chaque dispositif de surveillance de l'anxiété.

12. Appareil selon l'une quelconque des revendications précédentes, comprenant par ailleurs une bibliothèque d'ensembles de scènes, chaque ensemble de différents scénarios ou un niveau différent de défi pour le même scénario.

13. Appareil selon la revendication 12, dans lequel la bibliothèque d'ensembles de scènes comprend par ailleurs un ensemble de scènes qui sont configurées pour amener l'individu recevant la thérapie à se détendre.

14. Appareil selon l'une quelconque des revendications précédentes, dans lequel l'appareil comporte par ailleurs un écran d'affichage situé à l'extérieur de l'enceinte et dans lequel la caméra située à l'intérieur de l'enceinte et configurée pour capturer le comportement du patient et du thérapeute au cours de l'utilisation de l'appareil est connectée à l'écran d'affichage situé à l'extérieur de l'enceinte à des fins d'affichage d'images capturées par ladite caméra sur ledit écran d'affichage en temps réel.
